# EUROPEAN PATENT APPLICATION

(11) **EP 4 360 555 A1**
(43) Date of publication of application: **01.05.2024**
(21) Application number: 22203711.1
(22) Date of filing: 26.10.2022
(51) Int. Cl.: A61B 6/00, A61B 6/10

(54) **A DEVICE, A METHOD AND AN X-RAY SYSTEM FOR LOCKING AND UNLOCKING A POSITION OF AN X-RAY TUBE HEAD**

(71) Applicant: Koninklijke Philips N.V., 5656 AG Eindhoven (NL)
(72) Inventor: DUTT, Tara, 5656AG Eindhoven (NL)
(74) Representative: Philips Intellectual Property & Standards

(57) **Abstract**

The invention concerns a device and a method, and an X-ray system comprising such a device, comprising a first contact element configured to engage with a first engagement element, a second contact element configured to engage with a second engagement element, a cam element which is movably provided, an actuator and a spring attached to the cam element, wherein the cam element is arranged between the actuator and the spring. The first contact element and the second contact element are configured for being movable by the cam element, wherein the first contact element and the second contact element are configured for being movable between a locking positon and an unlocking position, wherein in the locking position the first contact element engages with the first engagement element and the second contact element engages with the second engagement element; wherein in the unlocking position the first contact element is spaced apart from the first engagement element and the second contact element is spaced apart from the second engagement element,. The actuator is configured upon activation to rotate the cam element such that the cam element is configured to move the first and the second contact element from the locking position to the unlocking position.

## Description

### FIELD OF THE INVENTION

The invention relates to the field of X-ray acquisition, in particular to the field of positioning an X-ray tube head and a locking of the position of the X-ray tube head for an X-ray acquisition. More specifically, the invention relates to a device and a method for locking and unlocking a position of an X-ray tube head and an X-ray system comprising such a device.

### BACKGROUND OF THE INVENTION

In practice an X-ray system, may be attached to a ceiling and comprises a ceiling suspended X-ray holding system which consists of a carriage that moves in horizontal plane along the ceiling via X-axis and Y-axis mechanism provided on ceiling rails. Further, the system comprises a telescope column, which is attached to the carriage. The telescope column can be extended via a Z-axis mechanism, also mounted on the carriage. The lower portion of the telescope column carries the X-ray tube head. The X-ray tube head comprises an X-ray tube, a collimator, an X-ray operation panel mounted at the front of the X-ray tube device and a base handle for moving the X-ray tube head. The X-ray tube head can be moved along a swing axis and a tilt axis with respect to the telescope column. A swing axis mechanism is mounted below telescopic column and the tilt axis is perpendicular to swing axis in the plane of telescopic columns cross sections. The X-ray system is equipped with a locking mechanism that fixes the axis movement. Each of the axis mechanism used for moving the X-ray device and/or the X-ray tube head are locked by a respective locking mechanism. The used locking mechanism comprises large parts assembled together and these mechanisms have a limited life span due to wear and durability. Further, the present locking mechanism are very complex and bulky which causes many field issues of the X-ray tube positioning due to slippage or rotation.

### SUMMARY OF THE INVENTION

There exists a need for optimizing the locking mechanism for position an X-ray tube head. In particular, there exist a need for an improved assembly, such that the size of the part to be used for the locking device can be reduced. Hence, the manufacturing is eased and the cost is decreased. The object of the present invention is solved with the subject matter of the independent claims, wherein further embodiments are incorporated in the dependent claims.

An object of the invention is to provide an improved locking device for locking and unlocking a position of an X-ray tube head such that the locking device comprise a longer durability, i.e. holds longer, is easy to manufacture and is reduced in size. Moreover, the device provides a locking, which comprises an improved friction and slippage behavior, in particular during rotating the X-ray tube head and/or during adjusting of the locking and unlocking position.

It should be noted that any feature, function and/or element described in the following with reference to the device equally applies to the method, and/or the X-ray system comprising the device, and vice versa. Accordingly, any feature, function, step and/or element described in the following with reference to one aspect of the present disclosure equally applies to any other aspect of the present disclosure.

According to a first aspect of the invention a device for locking and unlocking a position of an X-ray tube head comprises a first engagement element and a second engagement element, a first contact element configured to engage with the first engagement element, a second contact element configured to engage with the second engagement element, a cam element which is movably provided, wherein the first and the second contact element are attached at opposite positions on a same surface of the cam element, an actuator and a spring attached to the cam element, wherein the cam element is arranged between the actuator and the spring. The first contact element and the second contact element are configured for being movable by the cam element, wherein the first contact element and the second contact element are configured for being movable between a locking positon and an unlocking position. In the locking position, the first contact element engages with the first engagement element and the second contact element engages with the second engagement element. In the unlocking position, the first contact element is spaced apart from the first engagement element and the second contact element is spaced apart from the second engagement element. In this embodiment, the actuator is configured upon activation to rotate the cam element such that the cam element is configured to move the first contact element and the second contact element from the locking position to the unlocking position, whereby the spring is configured to move due to the rotation of the cam element. In particular, the spring is configured and arranged in such a manner, that it follow a movement of the cam element when the actuator moves the cam element. Further, the spring is configured, upon deactivation of the actuator, to rotate the cam element due to a reset force of the spring such that the cam element is configured to move the first contact element and the second contact element from the unlocking position to the locking position. In other words, when the actuator is deactivated, the movement of the cam element is generated by the spring, which is urged into its normal state by the reset force of the spring.

In the context of the present invention, the term "engagement element" shall be understood to describe a mechanical part of the device with which another part may form a connection during an attachment, when a contact is formed. The connection, i.e. the engagement, may be formed in a releasable manner, such that the connection may not be permanent and can be removed.

In the context of the present invention, the term "movably provided" shall be understood to describe, that the cam element may be moved by any other part, which is arranged and configured for moving the cam element. In particular, it describes that the attachment of the cam element at the device is provided in such a manner that the cam element is not fixed at its attachment position. Instead, the cam element comprises a structural attachment allowing a movement, in particular along its center axis. Accordingly, the cam element may be movable by another part of the device. In particular, the cam element may move or may stand still, i.e. rest on a position and is not moving.

In the context of the present invention, the term "actuator" shall be understood to describe, any part and/or element, which could be used for activating a movement of the cam element. In particular, the actuator may be any kind of element, which can be attached with/at the cam element, such that the cam element can be moved by the actuator. The actuator may be activated electrically and/or manually by the device, the X-ray system and/or the user, wherein also a combined activation of the mentioned electrically and manually activation may be possible.

In the context of the present invention, the term "locking position" shall be understood to describe a position of the device, wherein the elements of the device are arranged in such a manner that the position of the X-ray tube head is locked and the X-ray tube head cannot be moved any more. Moreover, the term "locking position" shall be understood to describe a position of the parts of the device in which these parts are also locked and cannot be moved any more.

Contrary, the term "unlocking position" shall be understand to describe, any position of the device wherein the elements of the device are arranged in such a manner that the position of the X-ray tube head can be changed, which means that the X-ray tube head may be moved, or is movable. Moreover, the term "unlocking position" shall be understood to describe a position of the parts of the device in which these parts are also unlocked and can be moved.

The locking and/or unlocking position may not be limited to a particular position. The locking and unlocking position may be any position of the elements and/or parts allowed by the device. In particular, the locking/unlocking position may be any position to which the cam element can be rotated. Viewed from the position of the cam element, the cam rotation may cause the locking and the unlocking within a rotation around an angle of 45° between the locking position and the unlocking position. The angle difference between the two position may be in such a manner that it is sufficient that the cam element rotation may cause the contact element(s) to be fully retracted. The locking and/or unlocking position may be any position, which the X-ray tube head should take, i.e. the X-ray tube head may be positioned to.

It should be noted, that for a better understanding some embodiments may be described by referring to only one contact element, nevertheless the described features, only described with respect to the first contact and/or the first engagement element may also apply in a similar manner to the second contact and/or the second engagement element and vice versa.

The structural members of this invention are the actuator, the cam element, the first and second engagement element and the first and second contact element and the spring. The device achieves two positions for locking and unlocking, wherein these positions in general may be achieved due to the movement of the actuator, and wherein this movement of the actuator causes to move other structural elements of the device for arranging the device, hence parts thereof, in a locking or unlocking position. In general, the two positions does not mean, that the device is limited to two spatial positions, it shall be understood do describe that the mechanical elements of the device can achieve a locking and unlocking, wherein for this states the mechanical elements are arranged at specific positions to each other. In particular, the X-ray tube head, which may be equipped with such a device, is able to rotate around any angle, in particular around + 180° and -180°, and the device is able to lock the position of the X-ray tube head at any desired angle. This invention allows that the device provides a flexibility for the X-ray tube head in such a manner that the X-ray tube head assembly can be rotated around +180° and around -180°. For instance, if the X-ray tube head and the device attached thereto is arranged in an X-ray tube head system arranged at a ceiling suspended holding system, the X-ray tube head may be rotatable around +180° and around -180° of a telescopic column of the holding system.

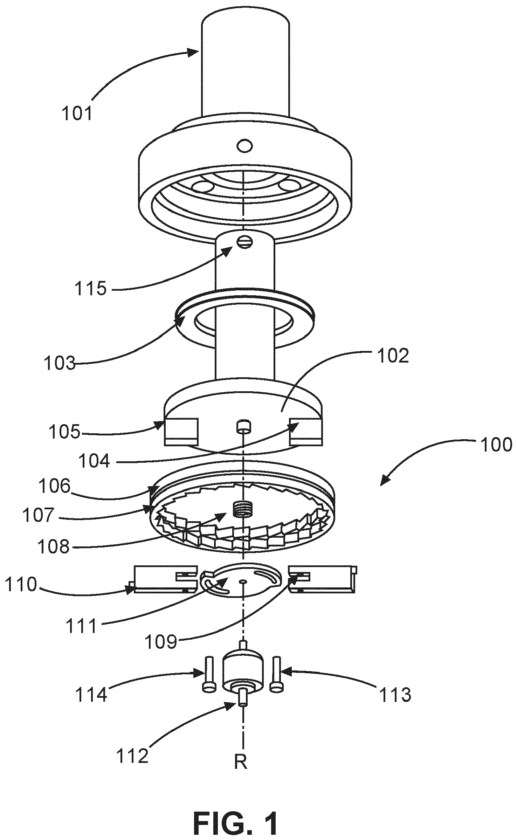

In this embodiment, in particular when the actuator is activated, the cam element may be configured and arranged to be rotatable around the rotational axis of the cam element, wherein the rotational axis corresponds to the center axis of the cam element. When the cam element is rotated the contact element follows the cam element, further details may be described in the following embodiments and the Fig. 1 to 4. The first and/or second contact element rests, or stops, at the engagement element and abuts at the engagement element in the locking position, wherein in the unlocking position the contact element(s) are spaced apart from the engagement element(s). The activation of the actuator results in a movement of the contact element(s), wherein it is disengaged from the engagement element(s), hence moved away from the engagement element(s). In other words, the contact element(s) may be retracted by the cam element and an unlocking occurs. On the other hand, when deactivating the actuator the spring pushes the contact element(s) back (by moving, e.g. pushing, back the cam element) and towards the engagement element(s) and the device is brought into the locking position, which may also be called a locking state, locking condition.

According to a further embodiment, the first engagement element and the second engagement element are arranged in a stacked manner above each other. Accordingly, the first contact element may be configured and formed in such a manner that it is only able to engage with the first engagement element and the second contact element may be configured and formed in such a manner that it only is able to engage with the second engagement element. According to this configuration, the device may be able to provide a locking and unlocking in a clockwise and counterclockwise direction, hence in a backward and forward rotation direction along, for instance, a horizontal plane of the X-ray tube head.

In general, the parts of the device as mentioned hereinabove may be centered at one and the same axis, which axis may be the rotational axis of the cam element, i.e. the center axis of the cam element. This means, the actuator the cam element, the spring and the first and second engagement elements may be centered at the rotational axis, hence are arranged with their center axis at the same rotational axis. Further, the actuator may be attached to the cam element such that a bolt/pin of the actuator extends through an opening of the cam element, wherein this opening may be formed at the center of the cam element. In particular, the opening at the center of the cam element may be a through hole.

According to an exemplary embodiment of the invention, the first contact element may be attached to the cam element by a first guiding element extending through a first slot formed at the cam surface, wherein the second contact element is attached to the cam element by a second guiding element extending through a second slot formed at the cam surface. The slot(s) formed at the cam element may be an elongated through hole through which the respective guiding element may extend. Hence, the guiding element(s) can be moved inside the slot(s). Due to the attachment of the contact element(s) at the guiding element(s), the contact element(s) is able to follow a movement of the cam element. Therefore, the cam element may transfer the movement around the center axis of the cam element via the guiding element to the contact element(s), such that the contact element(s) can be moved between a locking and unlocking position, for instance backward and forward in a direction perpendicular to the center axis of the cam element. The contact element(s) is fixedly attached to the guiding element(s), for instance, these elements are fixed permanently to each other.

According to an exemplary embodiment, the first guiding element and/ or the second guiding element is at least one of a screw, a pin, or a bolt. The guiding element may be attached the respective contact element (the first and/or the second contact element) in a fixed manner to the cam element, such that when the cam element is moved also one or each of the contact element(s) is moved directly, and follows a movement of the cam element.

According to an exemplary embodiment of the invention, the first contact element and/or the second contact element may be configured for providing a positive locking with the first engagement element and/or the second engagement element. In other words, the device may be configured to provide a form-locking (positive locking) between the one or more contact element and the respective engagement element. Accordingly, the first contact element may be engaged with the engagement element in the locking position in a form-fitting manner, wherein the shape of the contact element is adapted to the shape of the engagement element. In particular, the shape of the contact element (first and/or second contact element) may be the positive form and the respective engagement element (first and/or second) may comprise the respective negative form. Therefore, the device is able to provide a positive locking for positioning the X-ray tube head at all angles for an X-ray imaging, hence for a radiographic application.

According to an exemplary embodiment of the invention, the cam surface of the cam element may extend in a plane perpendicular to a rotational axis of the cam element. The first slot may extend on the cam surface in circumferential direction around the rotational axis of the cam element and wherein the second slot may be formed at the cam surface opposite to the first slot, wherein the second slot may extend in circumferential direction around the rotational axis of the cam element. The first guiding element may be arranged inside the first slot and may be configured for guiding the first contact element along the first slot during the movement of the cam element. The second guiding element may be arranged inside the second slot and may be configured for guiding the second contact element along the second slot during the movement of the cam element. In other words, each slots may comprise a shape forming a so-called slotted profile, wherein the contact element may follow the slotted profile due to the respective guiding element(s) arranged inside the slot.

According to an exemplary embodiment of the invention, the cam element may be configured to pull each of the first and the second contact element from the locking position into the unlocking position during the rotation of the cam element. The cam element may be further configured to push each of the first and the second contact element from the unlocking position into the locking position during the rotation of the cam element. In other words, the contact elements are arranged at the cam element in such a manner that due to a movement, for instance a rotation around the center axis, of the cam element, the contact elements are pushed away from the center axis of the cam element along a direction perpendicular to the center axis of the cam element and thus bringing the device in a locking position. When the contact element(s) is pushed as far as possible the contact element engages with the engagement element(s). When the contact elements are pulled, the contact elements are moved, e.g. pulled, into the direction of the center axis of the cam element such that the contact elements are configured to move along a plane perpendicular to the center axis of the cam element into the direction of the center axis of the cam element. Accordingly, the engagement is released and the contact elements are spaced apart from the engagement elements.

According to an exemplary embodiment of the invention, the contact element may comprise a rectangular shape. For instance, each of the contact elements may be a rectangular bar. The bar extends along a plane perpendicular to the rotational axis of the cam element. A first side of the bar, the contact element, is attached to the cam element. The opposite side of the bar is configured and shaped for being engaged with the respective engagement element. In particular, the opposite side extends over the cam element into a direction away from the rotational axis of the cam element. For the guiding element, at the bar a through hole may be formed at the side of the bar, which is attached to the cam element, such that the guiding element can be inserted into this through hole for attaching the contact element, the bar, at the cam element. For instance, the guiding element is fixedly attached to the contact element but movably provided at the cam element. This embodiment may apply to all contact element and/or guiding elements as described in the other embodiments.

According to an exemplary embodiment of the invention, the first slot may comprise an elongated shape having a first end and a second end between which ends the first slot is formed. Further, a center of the first end of the first slot may be arranged at a first radius extending along the circumferential direction from the rotational axis of the cam element, wherein a center of the second end of the first slot may be arranged at a second radius extending along the circumferential direction from the rotational axis of the cam element. Further and/or additionally, the first radius of the first slot may be larger than the second radius of the first slot. Also, the second slot may comprise an elongated shape having a first end and a second end between which ends the second slot is formed. Further, a center of the first end of the second slot may be arranged at a first radius extending along the circumferential direction from the rotational axis of the cam element, wherein a center of the second end of the second slot is arranged at a second radius extending along the circumferential direction from the rotational axis of the cam element. Further and/or additionally the first radius of the second slot may be larger than the second radius of the second slot. The slotted profile of the cam element may be shaped as described hereinabove. In particular, the slotted profile may extend on the cam surface along the circumferential direction at a variable distance (x) from the center axis of the cam element, due to the different radius of the ends of each respective slot. The total travel of the contact element along a plane perpendicular to the rotational axis inside the slots (by the guiding element), may be equal to the difference between the first radius and the second radius. This feature may also apply to the first slot and the second slot.

According to an exemplary embodiment of the invention, the actuator may be a rotary solenoid comprising a center axis arranged at a center of the cam element. The rotary solenoid may be actuated electrically and/or manually. In general, the actuator may be actuated electrically and/or manually. As alternative embodiments the actuator may be any element which his able to move the cam element in such a manner that it is able to move and/or transfer the first and second cam element between a locking position and a unlocking position and vice versa. For instance, the actuator may be a rotary magnet, an electrical motor etc.

According to an exemplary embodiment of the invention, the spring may be a torsional spring. Further, additionally, the spring may be attached to the cam element through an opening in the cam element, wherein the opening is spaced apart from the rotational axis of the cam element. Alternatively the spring may also be attached in a different manner to the cam element, for instance the spring may be attached to a surface of the cam element, which surface faces the spring, which surface is the opposite surface to the surface facing the contact elements. The spring may be attached to the cam element by welding. On the other hand, the spring may be attached to the cam element by any other structural element which can be attached to the cam element and which is configured for receiving a part of the spring. By attaching the spring to the cam element the cam element may be forced by a reset force of the spring to move, in particular when the actuator is deactivated.

According to an exemplary embodiment of the invention, the device may further comprise a housing arranged and configured for attaching the device to the X-ray tube head. The first and second engagement element and the first and second contact element, the cam element, the actuator and the spring may be arranged inside of the housing. Further, a rotating disk may be arranged in the housing, wherein the rotating disk may be arranged at a surface of the cam element opposite to the surface at which the first and second contact element is arranged. Furthermore, the rotating disk is configured and arranged for supporting the cam element and the first contact element and the second contact element when being moved. The rotating disk may support the movement of one or each of the contact element(s). In particular, at the rotating disk a first support element may be arranged at which the first contact element is movably arranged. Further, a second support element may be arranged at which the second contact element may be movably arranged. Moreover, the support element(s) may have a shape of a guiding rail, such that the contact element(s) may be arranged with one side (the side, or surface, facing the cam element) in the guiding rail and are able to move forward and backwards. In the context of this embodiment, the forward movement shall be understood to describe a movement from the contact element (first and/or second contact element) away from the rotational (center) axis of the cam element on a plane perpendicular to the rotational axis, hence a movement into the direction of the engagement element(s) for engagement with the engagement element(s). The backward movement may be a movement in the opposite direction, hence into the direction of the rotational (center) axis of the cam element, away from the engagement element(s).

According to an exemplary embodiment of the invention, the rotating disk may comprise a plate and a shaft, wherein the shaft extends into the direction of the housing, such that the shaft may be configured for being attached to the X-ray tube head and the housing. Further, the plate may be the main disk element, wherein the plate may be arranged at a surface of the cam element opposite to the surface at which the first and second contact element is arranged. Further, at the plate, the first support element and the second support element may be attached at a surface of the plate facing the cam element and wherein this surface (of the plate) is the surface of the plate opposite to the shaft.

According to an exemplary embodiment of the invention, the first engagement element may comprise a ring shape, along which inner circumferential surface a first plurality of recesses is arranged. Further, also the second engagement element may comprise a ring shape, along which inner circumferential surface a second plurality of recesses is arranged. In particular, both of the first and the second engagement elements are centered at the center axis of the cam element. The first contact element may engage in the locking position with at least one of the first plurality of recesses and the second contact element may engage in the locking position with at least one of the second plurality of recesses. In particular, the recesses may comprise a toothed shape, such that the engagement element (the first and the second) may be a toothed ring, toothed wheel. The size of the recesses of the engagement element(s) is adapted to the size of the contact element, such that a form-fitted, a positive locking, engagement can be formed.

According to an exemplary embodiment of the invention, the first contact element may comprise an end having a first protrusion, which is configured to engage with the first engagement element and another end, which is in contact with the cam element. Further, the first protrusion may extend into the radial direction of the center axis of the cam element along a plane perpendicular to the center axis. Furthermore, the second contact element may comprise an end having a second protrusion, which is configured to engage with the second engagement element and another end, which is in contact with the cam element. Further, the second protrusion extends in radial direction of the center axis of the cam element along a plane perpendicular to the center axis. In particular, the first and/or second protrusion at the respective contact element may be formed in such a manner that they comprise a shape formed such that it fits into the respective recess of the engagement element(s). For example, the first and/or second protrusion may be a tooth extending away from the rotational axis from the cam center and the tooth protrusion may fit into a toothed recess of the engagement element(s). The size of the recesses of the engagement element(s) is adapted to the size of the protrusions of the contact element(s), such that a form-fitted, a positive locking, engagement can be formed. In particular, the size of the first protrusion is adapted to the size of the plurality of first recesses and the size of the second protrusion is adapted to the size of the plurality of second recesses.

According to an exemplary embodiment of the invention, the first protrusion may have a triangle shape and wherein the first engagement element has a corresponding triangle shape configured for engaging with the first protrusion, wherein the second protrusion has a triangle shape and wherein the second engagement element has a corresponding triangle shape configured for engaging with the second protrusion. According to this embodiment, the protrusion may comprise a tooth having a triangle shape and the recesses at the engagement element(s) may comprise a toothed shape, such that they form a plurality of teeth with triangle shape.

According to an exemplary embodiment of the invention, the device may be configured for locking a position of the X-ray tube in a horizontal plane and/or in the vertical plane. In particular, the device may be configured for locking in the horizontal plane into two different directions, for instance in the clockwise and counter clockwise direction into which the X-ray tube head may be rotated.

According to a second aspect of the invention a method for locking and unlocking a position of an X-ray tube head comprises the following steps. Providing a cam element movable by an actuator around a center axis of the cam element. Moving a first contact element by the cam element upon movement of the cam element, between a locking position and an unlocking position. Moving a second contact element by the cam element upon movement of the cam element, between a locking position and an unlocking position. Engaging the first contact element with the first engagement element in the locking position. Engaging the second contact element with the second engagement element in the locking position. Releasing the first contact element from the first engagement element when moving from the locking position to the unlocking position. Releasing the second contact element from the second engagement element when moving from the locking position to the unlocking position. Activating the actuator for rotating the cam element such that the cam element moves the first and second contact elements from the locking position to the unlocking position, whereby a spring arranged at the cam element, is moved due to the rotation of the cam element. A further step may be deactivating the actuator such that the movement of the cam element is stopped and such that the spring rotates the cam element due to a reset force of the spring such that the cam element moves the first contact element and the second contact element from the unlocking position to the locking position. The order of the steps may not be limited to the order as described above or as described in any other embodiment of the invention. Accordingly, the order of the steps may be changed, wherein only one step may be exchanged with another, or more than one step may be exchanged by more than one another step.

In this embodiment, the actuator may rotate the cam element, such that the cam element moves the first and second contact element between the locking position and the unlocking position.

According, to another embodiment of the invention, an X-ray tube head may comprise an X-ray tube, an operation panel, a handle configured for being gripped by a user for moving the X-ray tube head, a swing mechanism configured for moving the X-ray tube head in a horizontal plane, and a device according to any of the embodiments described hereinabove for locking and unlocking a position of the X-ray tube head adjusted by the swing mechanism.

According to a third aspect of the invention an X-ray system comprises an X-ray tube head, an X-ray tube head positioning apparatus, and a device according to any of the embodiments of the present invention. The device is configured for locking and unlocking a position of the X-ray tube head adjusted by the positioning apparatus. The device provides the freedom to the tube head to rotate in the horizontal plane and tilt along the tilting axis, such that the tube head may take different positions for radiographic imaging. In particular, the position of the X-ray tube may be locked and/or unlocked at any angle of +180° and around -180° by the device according to any one of the embodiments of the invention described herein.

According to an exemplary embodiment of the invention, the X-ray system may further comprise a ceiling, a carriage and a telescope column. The carriage may be movably attached to the ceiling for being moved along the ceiling along a horizontal plane. An upper end of the telescope column is attached to the carriage and wherein the telescope column is extendable along an axis in a direction away from the ceiling, wherein the axis is perpendicular to the horizontal plane. The X-ray tube head is attached to a lower end of the telescope column, wherein the X-ray tube head is rotatable along a horizontal plane and a vertical plane at the lower end of the telescope column.

It has to be noted that embodiments of the invention have been described with reference to different subject matters. In particular, some embodiments have been described with reference to apparatus type claims whereas other embodiments have been described with reference to method type claims. However, a person skilled in the art will gather from the above and the following description that, unless other notified, in addition to any combination of features belonging to one type of subject matter also any combination between features relating to different subject matters, in particular between features of the apparatus type claims and features of the method type claims is considered as to be disclosed with this application.

### BRIEF DESCRIPTION OF THE DRAWINGS

Fig. 1 illustrates an exploded view of the device for locking and unlocking a position of an X-ray tube head according to an embodiment of the invention.
Fig. 2 illustrates a bottom view of the device in a locking position according to an embodiment of the invention.
Fig. 3 illustrates a bottom view of the device in an unlocking position according to an embodiment of the invention.
Fig. 4 illustrates a cam element of the device according to an embodiment of the invention.
Fig. 5 illustrates an X-ray system according to an embodiment of the invention.

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.

### DETAILED DESCRIPTION OF EMBODIMENTS

The illustrations in the drawings are schematic. It is noted that in different figures similar or identical elements are provided with the same reference signs.

In Fig. 1 an exploded view of the device 100 for locking and unlocking a position of an X-ray tube head according to an embodiment of the invention is shown. The device 100 comprises a first engagement element 106 and a second engagement element 107, a first contact element 109 configured to engage with the first engagement element 106, a second contact element 110 configured to engage with the second engagement element 107. The device 100 further comprises a cam element 111, which is movably provided, wherein the first and the second contact element 109, 110 are attached at opposite positions on a same surface 220 of the cam element 111. The device further comprises an actuator 112 and a spring 108 attached to the cam element 111, wherein the cam element 111 is arranged between the actuator 112 and the spring 108. The first contact element 109 and the second contact element 110 are configured for being movable by the cam element 111, wherein the first contact element 109 and the second contact element 110 are configured for being movable between a locking positon and an unlocking position. In the locking position, the first contact element 109 engages with the first engagement element 106 and the second contact element 110 engages with the second engagement element 107. Contrary, in the unlocking position, the first contact element 109 is spaced apart from the first engagement element 106 and the second contact element 110 is spaced apart from the second engagement element 107. The actuator 112 is configured upon activation, to rotate the cam element 111 around a central axis, which is also the rotational axis R of the cam element 111, such that the cam element 111 is configured to move the first and the second contact element 109, 110 from the locking position to the unlocking position, whereby the spring 108 is configured to move due to the rotation of the cam element 111. Further, the spring 108 is configured, upon deactivation of the actuator 112, to rotate the cam element 111 due to a reset force of the spring 108 such that the cam element 111 is configured to move the first contact element 109 and the second contact element 110 from the unlocking position to the locking position.

The first engagement element 106 and the second engagement element 107 may be arranged above each other, such that only the respective contact element 109, 110 can engage with the respective engagement element 106, 107. In particular, the first contact element 109 can only engage with the first engagement element 106 and the second contact element 110 can only engage with the second engagement element 107. The order of the engagement elements 106, 107 may also be changed, such that the first engagement element 106 may be arranged between the cam element 111 and the second engagement element 107, or vice versa.

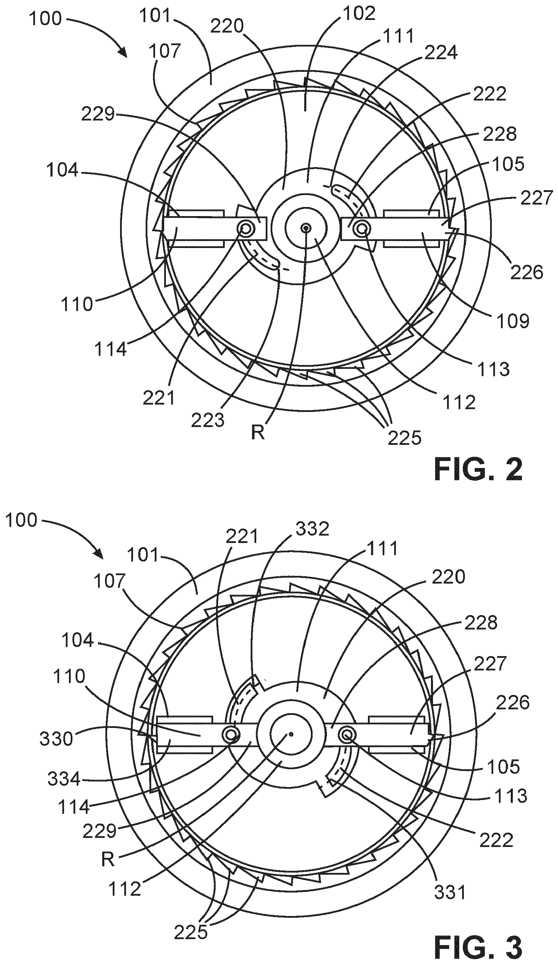

As shown in Fig. 1, the first contact element 109 is attached to the cam element 111 by a first guiding element 113 extending through a first slot 222 (illustrated in Fig. 2 and Fig. 3) formed at the cam surface 220. The second contact element 110 is also attached to the cam element 111 by a second guiding element 114 extending through a second slot 221 formed at the cam surface 220 (also illustrated in Fig. 2 and Fig.3).

The cam element 111 is configured to push each of the first and the second contact element 109, 110 from the locking position into the unlocking position during the rotation of the cam element 111 around the rotational axis R. On the other hand, the cam element 111 is configured to pull each of the first and the second contact element 109, 110 from the unlocking position into the locking position during the rotation of the cam element 111.

In Fig. 1, the illustrated actuator 112 is a rotary solenoid comprising a center axis arranged at a center of the cam element 111. In particular, the actuator 112 is arranged at the center axis of the cam element 111, such that the actuator 112 is able to rotate the cam element 111 around the center axis. For the attachment of the actuator 112 to the cam element 111 a connecting recess may be formed at the center of the cam element 111 into which recess the actuator 112 may be attached. The spring 108 is a torsional spring.

The device may further comprise a housing 101 arranged and configured for attaching the device 100 to the X-ray tube head of an X-ray system. The first and second engagement element 106, 107 and the first and second contact element 109, 110, the cam element 111, the actuator 112 and the spring 108 are arranged inside of the housing 101. Further, a rotating disk 102 is arranged in the housing 101, wherein the rotating disk 102 is arranged at a surface of the cam element opposite to the surface 220 at which the first and second contact element 109, 110 is arranged. The rotating disk 102 is configured and arranged for supporting the cam element 111 and the first contact element 109 and the second contact element 110 when being moved. Further, the rotating disk 102 is an integral part of the X-ray tube head, in particular of the X-ray tube mount, such that the movement of the X-ray tube head can be transferred to the device 100 and the device 100 can lock and unlock the position to which the X-ray tube head is moved.

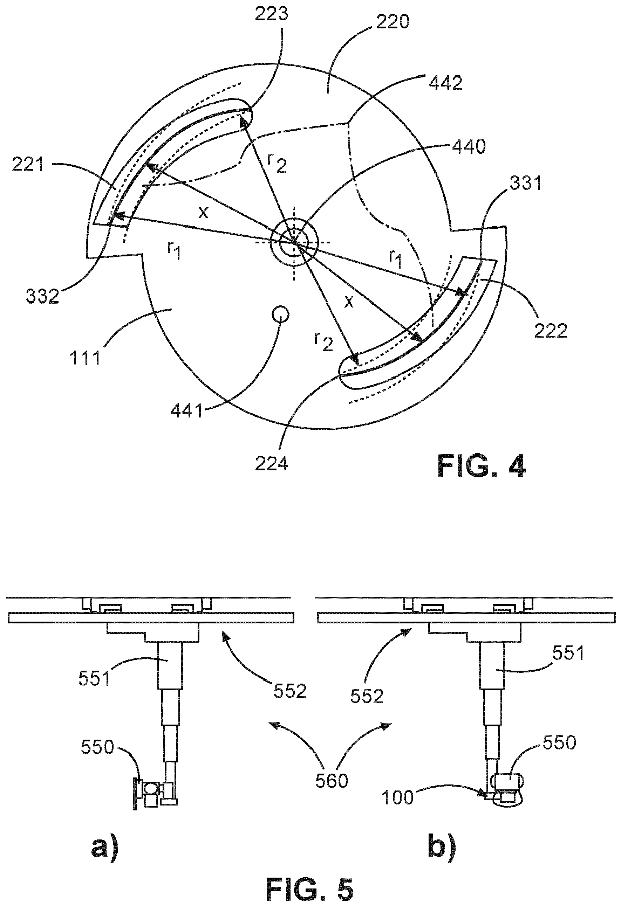

The parts of the device 100 are arranged at the same center axis, which is the rotational axis R. In particular, at least the actuator 112, the cam element 111, the spring 108, and the first and second engagement elements 106, 107 are arranged with their center at a common center axis, the rotational axis R. The cam element 111 rotates around this rotational axis R such that due to the attachment of the contact elements 109, 110 at the cam element 111, the contact elements 109, 110 are moved along a plane perpendicular to the rotational axis R in a direction away from the rotational axis R or into the direction of the rotational axis R. Also the housing 101 the disk 102 and its shaft 115 and a bearing, used for transmitting the rotation of the X-ray head, are arranged at a common center axis, which is the rotational axis. Further, the housing 101 maybe the part, which is connected to the X-ray system, in particular to the fixed, i.e. not moving part, element of the X-ray system, the telescope column (shown in Fig. 5).

In Fig. 2, a bottom view of the device 100 in a locking position according to an embodiment of the invention is shown. As can be seen in Fig. 2, and in the other Figures, the first engagement element 106 comprises a ring shape, along which inner circumferential surface a first plurality of recesses 225 is arranged. Hence, the first plurality of recesses extend from the inner surface of the engagement element 106 into the direction of the rotational axis R, wherein they in particular extend along a plane perpendicular to the rotational axis R. Further, also the second engagement element 107 comprises a ring shape, along which inner circumferential surface a second plurality of recesses (which is not visible in Fig. 2) is arranged. Hence, the second plurality of recesses extend from the inner surface of the engagement element 107 into the direction of the rotational axis R, wherein they in particular extend along a plane perpendicular to the rotational axis R. The recesses 225 of the first engagement element 106 and the recesses of the second engagement element 107 comprise a shape, which is similar. Nevertheless the direction into which the recesses 225 extend are opposite to each other (which is illustrated in Fig. 1 with the toothed rings and the plurality of teeth of the first engagement element 106 extend in a different direction than the plurality of teeth of the second engagement element 107). According to this, the locking can be carried out in both directions, hence in a clockwise and a counter clockwise direction.

The first contact element 109 engages in the locking position with at least one of the first plurality of recesses 225 and wherein the second contact element 110 engages in the locking position with at least one of the second plurality of recesses.

As illustrated in Fig. 2 the first contact element 109 comprises an end 227 having a first protrusion 226, which is configured to engage with the first engagement element 106 and another end 228, which is in contact with the cam element 111. The first protrusion 226 extends in radial direction of the center axis R of the cam element 111. The second contact element 110 comprises an end 334 having a second protrusion 330, which is configured to engage with the second engagement element 107 and another end 229, which is in contact with the cam element 111. The second protrusion 330 extends in radial direction of the center axis R of the cam element 111. In Fig. 2, the second protrusion 330 cannot be seen as it is engaged with the respective engagement element 106, which arranged, viewed from the bottom view of Fig. 2, below the second engagement element 107. Moreover, the first protrusion 226 has a triangle shape and wherein the first engagement element 106 has a corresponding triangle shape configured for engaging with the first protrusion 226. Similarly thereto, the second protrusion 330 has a triangle shape and wherein the second engagement element 107 has a corresponding triangle shape configured for engaging with the second protrusion 330.

The cam element 111 can be moved at any angle along the rotational axis R, wherein the attached contact elements 109, 110 can also be moved around the same angle along the rotational axis R. In particular the cam element moves from the locking position to the unlocking position within an angle of 45°.

Further, each the contact element 109, 110 is supported by a support element 104, 105. Wherein from the bottom view in Fig. 2 the support elements 104, 105 are arranged below the contact element 109, 110. The support elements 104, 105 are arranged at the disk 102. As can be seen the housing 101 surrounds all of the other parts of the device 100.

In this state as shown in Fig. 2 the actuator is deactivated, the cam element 111 is not rotated by the actuator 112. Instead, the spring 108 may has rotated the cam element 111 such that the contact elements 109, 110 are in the locking position at the engagement elements 106, 107.

In Fig. 3, a bottom view of the device 100 in an unlocking position according to an embodiment of the invention is shown. In Fig. 3 the same element of the device 100 as in Fig. 2 are shown. The difference between Fig. 2 and Fig. 3 is the position of the first and second contact element 109, 110. In Fig. 2 the contact elements 109, 110 engage with the respective engagement elements 106, 107 and in Fig. 3 the contact elements 109, 110 are spaced apart from the engagement elements 106, 107. In this state shown in Fig. 3 the actuator 112 is activated such that the cam element 111 is rotated and the contact elements 109, 110 are moved away from the engagement elements 106, 107.

In Fig. 4, a cam element 111 of the device 100 according to an embodiment of the invention is shown. As can be seen in Fig. 4, the cam surface 220 of the cam element 111 extends in a plane perpendicular to the rotational axis R of the cam element 111. Further, the first slot 222 extends in the circumferential direction around the rotational axis R of the cam element 111 and the second slot 221 is formed at the cam surface 220 opposite to the first slot 222. The second slot 221 extends in circumferential direction around the rotational axis R of the cam element 111. The first guiding element 113 can be arranged inside the first slot 222 and the first guiding element 113 is configured for guiding the first contact element 109 along the first slot 222 during the movement of the cam element 111. Further, the second guiding element 114 can be arranged inside the second slot 221 and the second guiding element 114 is configured for guiding the second contact element 110 along the second slot 221 during the movement of the cam element 111. As can be seen in Fig. 4, the first slot 222 comprises an elongated shape having a first end 224 and a second end 331 between which ends the first slot 222 is formed. A center of the first end 224 of the first slot 222 is arranged at a first radius r1 extending along the circumferential direction from the rotational axis R of the cam element 111, wherein a center of the second end 331 of the first slot 222 is arranged at a second radius r2 extending along the circumferential direction from the rotational axis R of the cam element 111. The first radius r1 of the first slot 222 is larger than the second radius r2 of the first slot 222. Due to this configuration the trajectory of the guiding elements 114, 113 inside the slots 221, 222 is realized. For instance, a follower profile 442 is illustrated in Fig. 4 showing the movement of the guiding elements 113, 114. The follower profile 442 extends inside of each slot 221, 222 from the center of the first end 223, 224 of the slot(s) 221, 222 to the center of the second end 332, 331 of the slot(s) 221, 222.

Also, the second slot 221 comprises an elongated shape having a first end 223 and a second end 332 between which ends the second slot 221 is formed. A center of the first end 223 of the second slot 221 is arranged at a first radius r1 extending along the circumferential direction from the rotational axis R of the cam element 111, wherein a center of the second end 332 of the second slot 221 is arranged at a second radius r1 extending along the circumferential direction from the rotational axis R of the cam element 111. The first radius r1 of the second slot 221 is larger than the second radius r2 of the second slot 221. In particular, the first radius r1 of the first slot 222 is equal to the first radius r1 of the second slot 221 and the second radius r2 of the first slot 222 is equal to the second radius r2 of the second slot 221. Accordingly, both contact elements 109, 110 will travel the same way guided by the guiding elements 113, 114 inside the slots 221, 222.

The spring 108 is attached to the cam element 111 through an opening 441 in the cam element 111, wherein the opening 441 is spaced apart from the rotational axis R of the cam element 111. In this opening 441, an arm of the spring 108 can be arranged in a fixed manner, such that the spring 108 is attached to the cam element 111.

In Fig. 5, an X-ray system 560 according to an embodiment of the invention is shown. The X-ray system 560 comprises an X-ray tube head 550, an X-ray tube head positioning apparatus (not visible), and a device 100 according to any of embodiments of the invention as described hereinabove. The device 100 is configured for locking and unlocking a position of the X-ray tube head 550 adjusted by the positioning apparatus. The X-ray system 560 further comprises a ceiling, a carriage 552 and a telescope column 551, wherein the carriage 552 is movably attached to the ceiling for being moved along the ceiling along a horizontal plane. An upper end of the telescope column 551 is attached to the carriage 552 and wherein the telescope column 551 is extendable along an axis in a direction away from the ceiling 552, wherein the axis is perpendicular to the horizontal plane. The X-ray tube head 550 is attached to a lower end of the telescope column 551, wherein the X-ray tube head 550 is rotatable along a horizontal plane and a vertical plane at the lower end of the telescope column 551. The device 100 is configured for locking a position of the X-ray tube head 550 in a horizontal plane and/or in the vertical plane.

Fig. 5 a) shows a first side view of the X-ray system 560 according to an embodiment of the invention, wherein the X-ray tube head 550 is rotated to a left side of the telescope column 551. The position of the X-ray tube head 550 is locked by the device 100 according to any one of the embodiments as described hereinabove.

Fig. 5b shows a side view of the X-ray system 560 as Fig. 5a, wherein the X-ray tube head 550 has been moved about 180° along the telescope column 551. Also, this position of the X-ray tube head 550 is locked by the device 100 according to any one of the embodiments as described hereinabove. For moving the X-ray tube head 550 from the position as shown in Fig. 5a the device 100 has been in the unlocked position, such that the X-ray tube head 550 is movable.

It should be noted that the term "comprising" does not exclude other elements or steps and "a" or "an" does not exclude a plurality. Also elements described in association with different embodiments may be combined. It should also be noted that reference signs in the claims should not be construed as limiting the scope of the claims.

### LIST OF REFERENCE SIGNS:

- 100: device
- 101: housing
- 102: disc
- 103: bearing
- 104: first support element
- 105: second support element
- 106: first engagement element
- 107: second engagement element
- 108: spring
- 109: first contact element
- 110: second contact element
- 111: cam element
- 112: actuator
- 113: first guiding element
- 114: second guiding element
- R: rotational axis
- 220: cam surface
- 221: second slot
- 222: first slot
- 223: first end of second slot
- 224: first end of first slot
- 225: recess
- 226: first protrusion
- 227, 228: end of first contact element
- 229, 334: end of second contact element
- 330: second protrusion
- 331: second end of first slot
- 332: second end of second slot
- 440: center axis of cam
- 441: opening
- 442: follower profile
- r1: first radius
- r2: second radius
- 550: X-ray tube head
- 551: telescope column
- 552: ceiling
- 560: X-ray system

## Claims

1. A device for locking and unlocking a position of an X-ray tube head, comprising
a first engagement element and a second engagement element,
a first contact element configured to engage with the first engagement element,
a second contact element configured to engage with the second engagement element,
a cam element, which is movably provided, wherein the first and the second contact element are attached at opposite positions on a same surface of the cam element,
an actuator and a spring attached to the cam element, wherein the cam element is arranged between the actuator and the spring,
wherein the first contact element and the second contact element are configured for being movable by the cam element,
wherein the first contact element and the second contact element are configured for being movable between a locking positon and an unlocking position,
wherein in the locking position the first contact element engages with the first engagement element and the second contact element engages with the second engagement element;
wherein in the unlocking position the first contact element is spaced apart from the first engagement element and the second contact element is spaced apart from the second engagement element,
wherein the actuator is configured upon activation to rotate the cam element such that the cam element is configured to move the first and the second contact element from the locking position to the unlocking position, whereby the spring is configured to move due to the rotation of the cam element,
wherein the spring is configured, upon deactivation of the actuator, to rotate the cam element due to a reset force of the spring such that the cam element is configured to move the first contact element and the second contact element from the unlocking position to the locking position.

2. The device according to claim 1,
wherein the first contact element is attached to the cam element by a first guiding element extending through a first slot formed at the cam surface,
wherein the second contact element is attached to the cam element by a second guiding element extending through a second slot formed at the cam surface.

3. The device according to claim 2,
wherein the cam surface of the cam element extends in a plane perpendicular to a rotational axis of the cam element,
wherein the first slot extends in circumferential direction around the rotational axis of the cam element and
wherein the second slot is formed at the cam surface opposite to the first slot,
wherein the second slot extends in circumferential direction around the rotational axis of the cam element,
wherein the first guiding element is arranged inside the first slot and configured for guiding the first contact element along the first slot during the movement of the cam element, and
wherein the second guiding element is arranged inside the second slot and configured for guiding the second contact element along the second slot during the movement of the cam element.

4. The device according to any of the preceding claims,
wherein the cam element is configured to pull each of the first and the second contact element from the locking position into the unlocking position during the rotation of the cam element,
wherein the cam element is configured to push each of the first and the second contact element from the unlocking position into the locking position during the rotation of the cam element.

5. The device according to any of the claims 2 to 4,
wherein the first slot comprises an elongated shape having a first end and a second end between which ends the first slot is formed,
wherein a center of the first end of the first slot is arranged at a first radius extending along the circumferential direction from the rotational axis of the cam element, wherein a center of the second end of the first slot is arranged at a second radius extending along the circumferential direction from the rotational axis of the cam element,
wherein the first radius of the first slot is larger than the second radius of the first slot,
wherein the second slot comprises an elongated shape having a first end and a second end between which ends the second slot is formed,
wherein a center of the first end of the second slot is arranged at a first radius extending along the circumferential direction from the rotational axis of the cam element, wherein a center of the second end of the second slot is arranged at a second radius extending along the circumferential direction from the rotational axis of the cam element,
wherein the first radius of the second slot is larger than the second radius of the second slot.

6. The device according to any of the preceding claims,
wherein the actuator is a rotary solenoid comprising a center axis arranged at a center of the cam element,
wherein the rotary solenoid is actuated electrically and/or manually.

7. The device according to any of the preceding claims,
wherein the spring is a torsional spring,
wherein the spring is attached to the cam element through an opening in the cam element,
wherein the opening is spaced apart from the rotational axis of the cam element.

8. The device according to claim any of the preceding claims, further comprising
a housing arranged and configured for attaching the device to the X-ray tube head,
wherein the first and second engagement element and the first and second contact element, the cam element, the actuator and the spring are arranged inside of the housing,
a rotating disk arranged in the housing, wherein the rotating disk is arranged at a surface of the cam element opposite to the surface at which the first and second contact element is arranged,
wherein the rotating disk is configured and arranged for supporting the cam element and the first contact element and the second contact element when being moved.

9. The device according to any of the preceding claims,
wherein the first engagement element comprises a ring shape, along which inner circumferential surface a first plurality of recesses is arranged,
wherein the second engagement element comprises a ring shape, along which inner circumferential surface a second plurality of recesses is arranged,
wherein the first contact element engages in the locking position with at least one of the first plurality of recesses and wherein the second contact element engages in the locking position with at least one of the second plurality of recesses.

10. The device according to any of the preceding claims,
wherein the first contact element comprises an end having a first protrusion, which is configured to engage with the first engagement element and another end, which is in contact with the cam element,
wherein the first protrusion extends in radial direction of the center axis of the cam element,
wherein the second contact element comprises an end having a second protrusion, which is configured to engage with the second engagement element and another end, which is in contact with the cam element,
wherein the second protrusion extends in radial direction of the center axis of the cam element.

11. The device according to claim 11
wherein the first protrusion has an triangle shape and wherein the first engagement element has a corresponding triangle shape configured for engaging with the first protrusion,
wherein the second protrusion has a triangle shape and wherein the second engagement element has a corresponding triangle shape configured for engaging with the second protrusion.

12. The device according to any of the preceding claims,
wherein the device is configured for locking a position of the X-ray tube in a horizontal plane and/or in the vertical plane.

13. A method for locking and unlocking a position of an X-ray tube head, the method comprising the following steps of:
providing a cam element movable by an actuator around a center axis of the cam element,
moving a first contact element by the cam element upon movement of the cam element, between a locking position and an unlocking position,
moving a second contact element by the cam element upon movement of the cam element, between a locking position and an unlocking position,
engaging the first contact element with the first engagement element in the locking position,
engaging the second contact element with the second engagement element in the locking position,
releasing the first contact element from the first engagement element when moving from the locking position to the unlocking position,
releasing the second contact element from the second engagement element when moving from the locking position to the unlocking position,
activating the actuator for rotating the cam element such that the cam element moves the first and second contact elements from the locking position to the unlocking position, whereby a spring arranged at the cam element, is moved due to the rotation of the cam element.
deactivating the actuator such that the movement of the cam element is stopped and such that the spring rotates the cam element due to a reset force of the spring such that the cam element moves the first contact element and the second contact element from the unlocking position to the locking position.

14. An X-ray system comprising:
an X-ray tube head,
an X-ray tube head positioning apparatus,
a device according to any of the claims 1 to 12,
wherein the device is configured for locking and unlocking a position of the X-ray tube head adjusted by the positioning apparatus.

15. The X-ray system according to claim 14, further comprising:
a ceiling, a carriage and a telescope column,
wherein the carriage is movably attached to the ceiling for being moved along the ceiling along a horizontal plane,
wherein an upper end of the telescope column is attached to the carriage and wherein the telescope column is extendable along an axis in a direction away from the ceiling, wherein the axis is perpendicular to the horizontal plane,
wherein the X-ray tube head is attached to a lower end of the telescope column, wherein the X-ray tube head is rotatable along a horizontal plane and a vertical plane at the lower end of the telescope column.
